# NEW EUROPEAN PATENT SPECIFICATION

(11) **EP 0 494 599 B2**
(45) Date of publication and mention of the opposition decision: **13.10.1999**
(45) Mention of the grant of the patent: 16.04.1997
(21) Application number: 92100029.5
(22) Date of filing: 02.01.1992
(51) Int. Cl.: B01J 20/00, A61L 15/00, A61B 19/02, A61B 10/00, C02F 1/00, B65F 1/00

(54) **Use of gelling material and gelling method**
Verwendung von Geliermittel und Gelierungsverfahren
Utilisation de matière gélifiante et méthode de gélification

(30) Priority: 11.01.1991 JP 1391891; 30.01.1991 JP 3167991; 18.02.1991 JP 4605991
(43) Date of publication of application: 15.07.1992
(73) Proprietor: SANYO CHEMICAL INDUSTRIES, LTD., Kyoto-shi 605 (JP)
(72) Inventor: Fujiura, Yoji, Kyoto-shi, Kyoto-fu 607 (JP); Mita, Kouji, Yawata-shi Kyoto-fu 614 (JP); Shinoda, Katsumi, Otu-shi, Shiga-ken (JP)
(74) Representative: Kador & Partner

(56) References cited:
- EP-A- 0 278 601
- EP-A- 0 324 602
- WO-A-86/04680
- WO-A-90/00506
- FR-A- 1 475 399
- FR-A- 2 237 097
- FR-A- 2 368 994
- FR-A- 2 627 080
- GB-A- 2 017 107
- GB-A- 2 092 895
- JP-A- 2 157 083
- JP-Y- 63 010 800
- US-A- 4 102 842
- US-A- 4 335 722
- DATABASE WPIL Section Ch, Week 8707, Derwent Publications Ltd., London, GB; Class A97, AN 87-045460
- DATABASE WPIL Section Ch, Week 8911, Derwent Publications Ltd., London, GB; Class A96, AN 89-081142
- PATENT ABSTRACTS OF JAPAN vol. 1, no. 64 (C-017)22 June 1977
- DATABASE WPIL Section Ch, Week 8220, Derwent Publications Ltd., London, GB; Class A97, AN 82-40184
- Catalogue "Speciality paper" distributed by Mishima Paper Co.Ltd.
- Catalogue "Water-soluble paper series DISSOLVO" distributed by Mishima Paper Co., Ltd., cover page, reverse page thereof, back cover page.

## Description

### FIELD OF THE INVENTION

The present invention relates to use of gelling materials and gelling method useful for gelling aqueous fluids, medical wastes and body fluids collected by body fluid suction equipment. More specifically, the invention concerns use of gelling materials and gelling method for gelling various kind of aqueous fluids; body fluids such as blood and amniotic fluid, produced during operation in hospitals and parturition in maternity hospitals, medical wastes contaminated by body fluids, such as absorbent cotton, gauze, injection needles and operation gloves; and body fluids collected by body fluid suction equipment such as blood and amniotic fluid produced during operation and parturition.

### BACKGROUND OF THE INVENTION

Well-known gelling materials for gelling aqueous fluids include such absorbents as sand, bentonite, zeolite, sawdust, pulp, paper waste and water-absorbent resins.

Also, in many cases medical wastes produced in hospitals and maternity hospitals are collected in plastic or like containers for incineration therewith. Further, in many cases, body fluids collected by body fluid suction equipment in hospitals and maternity hospitals, are also incinerated together with their containers.

The above water-absorbents, however, have the following problems. (1) They are usually added in the form of powders or fine flakes, and at this time they may be spilled or produce dust. (2) Since they are in the form of powders or fine flakes, they require a lot of time for addition. (3) Their absorbing capacity is low, and therefore they have to be added in great quantities. (4) When pressure is applied to them after the absorption, they readily discharge the absorbed fluid to contaminate the surroundings.

Further, in the case of incinerating body fluids and other medical wastes directly without solidifying them, their containers may be occasionally broken during transportation to the incineration site, causing the wastes to flow out and soak into the ground. In such case, it is difficult to recover them, thus resulting in hazardous contamination of the ground.

Finally, US-A-4 335 722 disdoses a gelling material which comprises a core made of an absorbant material enclosed in a water-destroyable envelope, and an outer envelope. The outer enveloppe is not water-destroyable, so that the absorbant material of the core remains confined in this outer envelope.

### SUMMARY OF THE INVENTION

It is an object of the invention to provide a use of gelling material, and a gelling method, which permit ready handling without possibility of spilling or dusting and do not require conventional measuring or sprinkling time when adding the gelling material to aqueous fluids, medical wastes or body fluids in body fluid suction equipment.

It is another object of the invention to provide a use of gelling material, and a gelling method, which provide for high absorbing capacity, permit addition in small quantities, and ensure retention of fluid once absorbed even with application of external pressure to the gelling material after the absorption.

It is a further object of the invention to provide a use of gelling material, and a gelling method for medical wastes, which permit sale handling of medical wastes.

It is a still further object of the invention to provide a use of gelling material, and a gelling method for body fluids collected by body fluid suction equipment, which permit sale handling of body fluids recovered in containers in the body fluid suction equipment.

In order to accomplish the above objects, the invention relates to the use, for gelling aqueous fluids in a container, of a water-absorbent which is releasably contained in a wrapping comprising a laminated sheet which comprises water-disintegrating paper forming the outer surface of the wrapping and water-soluble film forming the inner surface of the wrapping, wherein said water-absorbent comprises a water-absorbing resin in the form of powders or granules, wherein the gelling material uses a filler together with the water-absorbing resin.

In its process aspects, the present invention relates to a method of gelling an aqueous fluid comprising adding an aqueous fluid gelling material to aqueous fluid, wherein said aqueous fluid gelling material comprises a water-absorbent which is releasably contained in a wrapping comprising a laminated sheet which comprises water-disintegrating paper forming the outer surface of the wrapping and water-soluble film forming the inner surface of the wrapping, and said water-absorbent comprises a water-absorbing resin in the form of powders or granules, wherein the gelling material uses a filler together with the water-absorbing resin.

Further, the present invention relates to gelling medical wastes by use of a water-absorbent which is releasably wrapped in a laminated sheet which comprises water-disintegrating paper forming the outer surface of the wrapping and water-soluble film forming the inner surface of the wrapping, wherein said water-absorbent comprises a water-absorbing resin in the form of powders or granules, wherein the gelling material for medical wastes uses a filler together with the water-absorbing resin.

In its process aspects, the present invention further provides a method of gelling medical wastes comprises adding a medical waste gelling material to a medical waste containing aqueous fluids, wherein said medical waste gelling material comprises a water-absorbent which is releasably wrapped in a laminated sheet which comprises water-disintegrating paper forming the outer surface of the wrapping and water-soluble film forming the inner surface of the wrapping, and said water-absorbent comprises a water-absorbing resin in the form of powders or granules, wherein the gelling material uses a filler together with the water-absorbing resin.

According to the invention, there is further provided use of a gelling material for body fluids collected by body fluid suction equipment, comprising a water-absorbent which is releasably wrapped in a laminated sheet which comprises water-disintegrating paper forming the outer surface of the wrapping and water-soluble film forming the inner surface of the wrapping, wherein said water-absorbent comprises a water-absorbing resin in the form of powders or granules, wherein the gelling material uses a filler together with the water-absorbing resin.

In its process aspects, the invention relates to a method of gelling fluid which flow into a disposable waste container set in a canister fixing container of body fluid suction equipment, which comprises adding a body fluid suction equipment gelling material to said fluid, wherein said body fluid suction equipment gelling material comprises a water-absorbent which is releasably wrapped in a laminated sheet which comprises water-disintegrating paper forming the outer surface of the wrapping and water-soluble film forming the inner surface of the wrapping, and said water-absorbent comprises a water-absorbing resin in the form of powders or granules, wherein the gelling material uses a filler together with the water-absorbing resin.

### BRIEF DESCRIPTION OF THE DRAWINGS

Figure 1 is a partially schematic sectional view showing a gelling material in one of the preferred embodiments of the invention; and
Figure 2 is a schematic sectional view showing a body fluid suction equipment including the gelling material in one embodiment of the invention.

### DETAILED DESCRIPTION OF THE INVENTION

According to the invention, as the water-absorbing resin there may be used ① those, which are obtainable by polymerizing (a) starch or cellulose, (b) a monomer containing a carboxyl group and/or a sulfonic acid group, the monomer being water-soluble or capable of being rendered water-soluble by hydrolysis, and (c) a crosslinking agent, as essential components, and carrying out hydrolysis, if necessary.

The details of the materials (a) to (c) used for the manufacture of the examples ① of the water-absorbing resin, proportions of the materials (a) to (c), method of manufacture and examples of the water-absorbing resin, are well known and disclosed in, for instance, Japanese published Patent Application (Kokai) No. 25886/1977, Japanese patent publication (Kokoku) No. 46199/1978, Japanese patent publication (Kokoku) No. 46200/1978 and Japanese patent publication (Kokoku) No. 21041/1980.

Other examples of the water-absorbing resin than those mentioned above are ② those obtainable by polymerizing the materials (a) and (b) (e.g., hydrolyzed starch-acrylonitrile graft-copolymer and hydrolyzed cellulose-acrylonitrile graft-copolymer); ③ those obtainable by crosslinking material (a) (e.g., crosslinked carboxymethyl cellulose); ④ copolymers of materials (b) and (c) (e.g., partially hydrolyzed crosslinked polyacrylamide, crosslinked acrylic acid-acrylamide copolymer, crosslinked sulfonated polystyrene, saponified vinylester-unsaturated carboxylic acid copolymer as disclosed in Japanese Published Patent Application (Kokai) No. 14689/1977 and Japanese Published Patent Application (Kokai) No. 27455/1977, crosslinked polyacrylic acid salt, crosslinked acrylic acid-acrylic acid ester copolymer, crosslinked isobutylene-maleic acid anhydride copolymer and crosslinked carboxylic acid denaturated polyvinyl alcohol; and ⑤ self-crosslinkable polymers of material (b) (e.g., self-crosslinkable polyacrylic acid salt). It is possible to use two or more kinds of the water-absorbing resin as disclosed above in combination.

Among the above examples of the water-absorbing resin, ① and some of ④ such as partially hydrolyzed crosslinked polyacrylamide, crosslinked acrylic acid-acrylic acid ester copolymer, crosslinked isobutylene-maleic acid anhydride copolymer, and crosslinked carboxylic acid denaturated polyvinyl alcohol are preferable.

The suitable water-absorbing resins in the above are those having an absorbing capacity with respect to pure water of at least 50 ml/g, preferably 100 to 1,000 ml/g. The resin is suitably in the form of powders or granules (with the particle size being usually within 5 mm).

If desired, other absorbents may be used in combination with the above mentioned water-absorbing resin (e.g., pulp and sawdust as well-known absorbents). However, the greater the proportion of the above mentioned water-absorbing resin, the more suitable.

According to the invention, a filler is used together with the absorbent. Examples of the filler are such inorganic fillers as activated clay, zeolite, talc, diatomaceous earth, bentonite, caryon, clay, silica gel, sand, pumice and gypsum and such organic fillers as sawdust, pulp, paper pieces and resin chips, thse fillers being in the form of particles, flakes, fibers, etc. By using these fillers together with the water-absorbing resin, it is possible to prevent agglomeration of the absorbent resin particles that might otherwise be caused when the absorbent is brought into contact with a body fluid or other aqueous fluid.

The amount of the filler to be used together with the absorbent according to the invention is usually 0.001 to 100 parts by weight, preferably 0.005 to 20 parts by weight, for one part by weight of the absorbent.

To the absorbent and/or filler according to the invention may be added deodorant, aromatic agent, bactericide, anti-rusting agent, antiseptics, anti-foaming agent, foaming agent, agglomerating agent, anti-blocking agent, surface active agent, deoxidation agent, extending agent, etc.

Examples of the water-disintegrating paper used according to the invention, are one which is obtained by bonding together paper pulp fibers with water-soluble or hydrophilic adhesive, water-swelling polymer, etc. such that the pulp fibers disintegrate when brought into contact with water (an example of such kind of paper is "Dissolvo WA" by Mishima Paper Mfg. Co., Ltd.), and one which is obtained by applying a heat seal agent to the above paper composition for providing forming processability (thermal bonding property) (an example of such kind of paper is "Dissolvo WAP" by Mishima Paper Mfg. Co., Ltd.). These examples of paper feature a high rate of disintegration when they absorb water.

Examples of the water-soluble film are water-soluble polyvinyl alcohol (poval) film, starch film, and carrageenan film. These films have higher tensile strength in dry state than that of the water-disintegrating paper noted above at the same thickness, although they are somewhat inferior in the speed of dissolution in water (i.e., disintegration).

As the laminated sheet obtainable by laminating the water-disintegrating paper and the water-soluble film, there may be used one which is obtained by bonding or laminating at least one kind of the water-disintegrating paper and the water-soluble film (e.g., "Dissolvo WAL" by Mishima Paper Mfg. Co., Ltd., obtained by laminating the poval film to "Dissolvo WA" noted above).

These laminated sheets are quickly degraded in water (i.e., quickly disintegrated) and also have high mechanical strength. Since the mechanical strength of paper is high, the thickness of the water-soluble film to be laminated can be reduced. Thus, both the degradation (or disintegration) speed and mechanical strength of the sheet can be increased.

The time required for the disintegration or dissolution of the wrapping material in water is usually 5 minutes or less, preferably 2 minutes or less, more preferably 1 minute or less.

According to the invention, the aqueous fluid may be any water-containing fluid and may contain such solids as sand, clay, gravel, inorganic oxides, hydroxides, rubber, plastic pieces, glass pieces, wood dust, fiber dust, carbon, carbohydrates, protein, solid fat, microorganisms, meat pieces and carcasses and liquids other than water, e.g., organic solvents, fatty oil, mineral oil, petroleum and active agents.

The water content in the fluid is usually 50 % or above. Examples of the aqueous fluid are sludge-like wastes produced in mining sites, civil and construction work sites, paper pulp manufacturing plants, fiber manufacturing and dying plants, machine and metal processing plants, mineral refining plants, chemical plants, electric and electronic product plants, medical industry facilities such as hospitals, food processing plants, ceramic product plants, water purification plants, sewage plants as well as homes.

Examples of the medical waste according to the invention are body fluids such as blood, lymph fluid, medullary fluid and amniotic fluid, and medical tools and consumables contaminated by these fluids, e.g., absorbent cotton, injection needles, operation gloves and disposable sheets. These medical wastes are produced during surgery in hospitals and parturition in maternity hospitals and may contain portions of removed body tissues, physiological salt solution having been used for washing of affected parts, water and antiseptic solution having been used for washing operation tools and tables, medicines, used ampules, dust, etc.

When the medical waste mainly consists of medical tools and consumables contaminated by the body fluids and contains little water, water may be added to it for effectively using the gelling material and gelling method according to the invention. With the method according to the invention, the medical waste which includes medical tools and consumables as noted above, may be converted into an integrated gel which may be readily and conveniently handled for disposal and also can prevent pollution and contamination of the surroundings.

According to the invention, the body fluids collected in the body fluid suction equipment refer to fluids in the living body as noted above and specifically blood, lymph liquid, medullary fluid, amniotic fluid, etc. These body fluids are produced during operation in hospitals and parturition in maternity hospitals. They may contain removed organism tissues, physiological salt solution having been used for washing of affected parts, water having been used for washing operation tools and tables, dust, etc.

The gelling material according to the invention may be manufactured by preliminarily producing a wrapping member having an opening having predetermined shape and size with any one of the wrapping materials noted above, then filling the wrapping member with a predetermined amount of the absorbent and a filler and then sealing the wrapping member by means of a heat seal, an adhesive, sewing, etc.

The gelling material for aqueous fluids according to the invention may have any shape such as bag-like, box-like, cylindrical, ball-like and spherical. It may also have any size. The gelling material for aqueous fluids may be used in amounts depending on the proportions of the water-absorbing resin and filler and also the character of the aqueous fluid to be processed. Usually, about one liter of ordinary aqueous fluid may be sufficiently gelled with 1 to 600 g of the gelling material according to the invention. If the amount of the gelling material is less than 1 g, the aqueous fluid can not be sufficiently gelled. An amount greater than 600 g, on the other hand, is uneconomical.

The aqueous fluid may be gelled in a short period of time (usually about one minute) by adding the gelling material according to the invention and suitably agitating the overall fluid. The agitation is carried out sufficiently to effect light mixing of the overall fluid. It may be done using a general-purpose agitator, which is not particularly limited. Manual agitation using a spatula is sufficient.

If solid articles such as injection needles are partly projecting from the fluid surface of medical waste or the like to be gelled, water may be added to the medical waste in the container before adding the gelling material according to the invention. In this way, the whole solid matter may be concealed with the gel.

Meanwhile, the gelling material according to the invention may be put into the container before medical waste such as body fluid is charged or flows into the container.

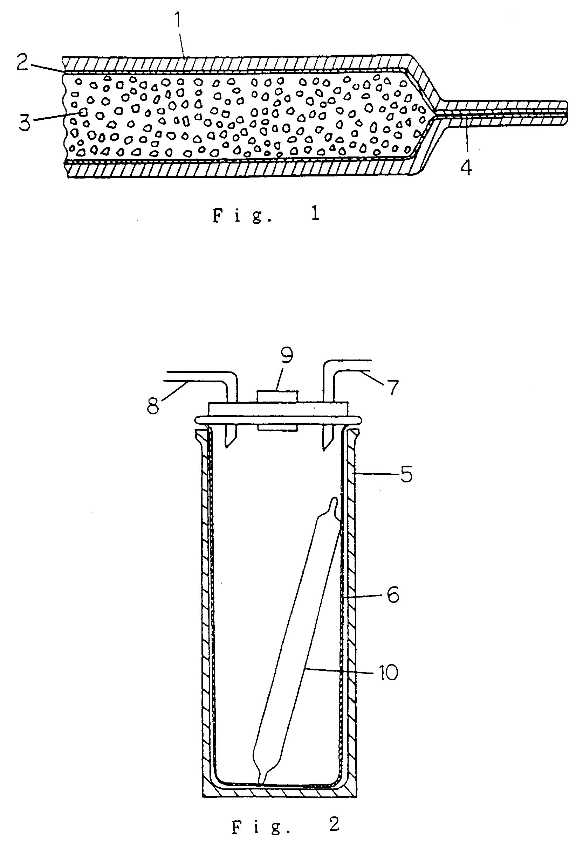

The invention will now be described with reference to the drawings. Figure 1 is a partially schematic sectional view showing one preferred embodiment of the gelling material according to the invention. Designated at 1 is water-disintegrating paper, at 2 is water-soluble polyvinyl ahcolol (poval) film bonded to the paper 1, at 3 is water-absorbing resin in the form of particles, and at 4 is a heat seal section. Although Figure 1 shows the gelling material in a partly broken-away view, the wrapping member is bag-like as a whole. The heat seal section is formed by heat sealing an opening, and it may be formed in at least one or all of the four sides, so long as the content is sealed in the wrapping member. Usually a heat seal agent is coated on the heat seal section.

Figure 2 is a schematic sectional view showing body fluid section equipment accommodating the gelling material according to the invention. Designated at 5 is a canister securement container, at 6 is a disposable container, at 7 is a body fluid inlet, at 8 is a body fluid outlet, at 9 is a gelling material inlet, and at 10 is a gelling material according to the invention. In this equipment, the gelling material 10 is preferably cylindrical, since this shape is convenient for charging the gelling material into the container. However, this shape is by no means limitative.

The body fluid gelling material for body fluid section equipment according to the invention may have a shape and a size such that it can be charged into the container used in the method according to the invention. As noted before, its shape may be bag-like, box-like, cylindrical, ball-like, spherical or any other shape. The amount of the water-absorbent for body fluid according to the invention depends on the character of the body fluid to be processed, but about 2 kg of body fluid, mainly comprising blood collected particularly during operation, may be sufficiently gelled with 5 to 100 g of the gelling material according to the invention.

According to the invention, the canister securement container 5 is usually made of plastics or metals, and its shape is usually cylindrical. It may have a structure that the disposable container 6 capable of accommodating body fluid withdrawn thereinto can be set in it. The disposable container 6 is not particularly limited so long as it can be set in the canister securement container and can hold the withdrawn body fluid. Usually, foldable beg-like disposable containers made of polyvinyl chloride, polyethylene, polypropylene. etc. are suitable for they are inexpensive and capable of ready handling.

The body fluid sucked into the disposable container may be gelled in a short period of time (usually about one minute) by adding the gelling material according to the invention and agitating the entire system. The agitation may be done to such an extent as to effect mixing of the system lightly. And circulating by continuous pouring of the fluid done is sufficient as agitation. To increase the efficiency of agitation, a general-purpose agitator may be used, although this is by no means limitative. Further, the gelling material according to the invention may be set in the disposable container before introducing the body fluid thereinto.

The invention will now be illustrated with reference to the following examples that by no means limit the scope of the invention.

### Example 1

A bag was produced by heat sealing along three edges of two overlapped oblong pieces 10 cm × 9 cm in size cut from "Dissolvo WAL" manufactured by Mishima Paper Mfg. Co., Ltd. This bag was then filled with 20 g of "SANWET IM-1000" (absorbing capacity : 1000 ml/g), an acrylic acid starch graft copolymer water-absorbing resin manufactured by Sanyo Chemical Industries, Ltd., and then its opening was heat sealed, thus obtaining a gelling material for aqueous fluids according to the invention.

This gelling material was added as such to 1.5 liters of sludge extracted from the sea bottom, and the resultant system was lightly agitated with a wooden spatula having a length of 20 cm and a width of 3 cm. The bag of the gelling material was disintegrated in about 10 secondes, and the overall system was gelled (i.e., solidified) in about 40 seconds. In this way, the sludge which was handled only with difficultly could be converted to solid readily capable of handling in a short period of time and conveniently.

### Comparative Example 1

A bag was produced by heat sealing along three edges of two overlapped oblong pieces 10 cm × 9 cm in size cut from "Dissolvo WA" manufactured by Mishima Paper Mfg. Co., Ltd. The bag was then filled with 20 of "SANWET IM-5000" (absorbing capacity : 400 ml/g), a partially crosslinked sodium polyacrylate manufactured by Sanyo Chemical Industries, Ltd., and then its opening was heat sealed, thus obtaining a gelling material for aqueous fluids according to the invention.

This gelling material was added to 1.5 liters of sludge-like paper pulp waste fluid, and the whole system was agitated with a wooden spatula having a length of 20 cm and a width of 3 cm. The bag of the gelling material was disintegrated in about 5 secondes, and the system was gelled (i.e., solidified) in about 30 seconds. Thus, the sludge which was handled only with difficulty could be converted to solid which could be readily handled.

### Comparative Example 2

A bag was produced by heat sealing along three edges of two overlapped oblong pieces 10 cm × 9 cm in size cut from a commercially available polyvinyl alcohol (poval) film. The bag was then filled with 20 g of partially crosslinked sodium acrylate-acrylamide copolymer (absorbing capacity : 250 ml/g), and then its opening was heat sealed to obtain a gelling material for aqueous fluids according to the invention.

This gelling material was added to 1.5 liters of raw sewage sludge, and the whole system was lightly agitated with a wooden spatula having a length of 20 cm and a width of 3 cm. The bag of the gelling material was disintegrated in about 40 secondes, and the system was gelled (i.e., solidfied) in about one minute. Thus, the sludge which was handled only with difficulty could be converted to solid which could be readily handled.

### Example 2

A bag was produced by heat sealing along three edges of two overlapped oblong pieces 10 cm × 9 cm cut in size from "Dissolvo WAL" manufactured by Mishima Paper Mfg. Co., Ltd. The bag was filled with "SANWET IM-1000" (absorbing capacity : 1000 ml/g), an acrylic acid starch graft copolymer water-absorbing resin manufactured by Sanyo Chemical Industries, Ltd., and its opening was heat sealed to obtain a gelling material for medical wastes according to the invention. In a 2-liter polyvinyl chloride container was extracted 1.5 liter of waste blood discharged during celiotomy, then the above gelling material was added as such, and the whole system was lightly agitated with a wooden spatula having a length of 20 cm and a width of 3 cm. The bag of the gelling material was disintegrated in about 10 secondes, and the system was gelled in about 50 seconds. Thus, blood in the form of fluid could be converted to solid, which could be readily handled, in a short period of time and conveniently.

### Comparative Example 3

A bag was produced by heat sealing along three edges of two overlapped oblong pieces 10 cm × 9 cm in size cut from "Dissolvo WA" manufactured by Mishima Paper Mfg. Co., Ltd. The bag was then filled with 20 g of "SANWET IM-5000" (absorbing capacity : 400 ml/g), partially crosslinked sodium polyacrylate manufactured by Sanyo Chemical Industries, Ltd., and then its opening was heat sealed, thus obtaining gelling material for medical wastes according to the invention.

1.5 liter of waste blood produced during operation containing about 50 wt. % of physiological salt solution used for washing was collected into a 2-liter polyvinyl chloride container . Then, the above gelling material was added and the system was lightly agitated with a wooden spatula having a length of 20 cm and a width of 3 cm. The bag of the gelling material was disintegrated in about 5 secondes, and the whole system was gelled in about 30 seconds. The blood in the form of fluid thus could be converted into solid capable of ready handling in a short period of time and conveniently.

### Comparative Example 4

A bag was produced by heat sealing along three edges of two overlapped oblong pieces 10 cm × 9 cm in size cut from a commercially available water-soluble polyvinyl alcohol (poval) film. The bag was then filled with 20 g of partially crosslinked sodium acrylate-acrylamide copolymer (absorbing capacity : 250 ml/g), and then its opening was heat sealed, thus obtaining a gelling material for medical wastes according to the invention.

1.5 liters of waste blood produced during operation containing about 50 wt. % of physiological salt solution used for washing was collected into a 2-liter polyvinyl chloride container. The above gelling material was then added and the system was lightly agitated with a wooden spatula having a length of 20 cm and a width of 3 cm. The bag of the gelling material was disintegrated in about 50 secondes, and the whole system was gelled in about 80 seconds. Thus, body fluid in the form of fluid could be converted into solid capable of ready handling in a short period of time and conveniently.

### Example 3

A bag was produced by heat sealing along three edges of two overlapped oblong pieces 10 cm × 9 cm in size cut from "Dissolvo WAL" manufactured by Mishima Paper Co., Ltd. The bag was then filled with 20 g of "SANWET IM-2200D" (absorbing capacity : 400 ml/g), an acrylic acid starch graft copolymer water-absorbing resin manufactured by Sanyo Chemical Industries, Ltd., and then its opening was heat sealed, thus obtaining a gelling material for medical wastes according to the invention.

3 liters of water was poured into a 5-liter vinyl chloride container containing medical wastes including a plurality of used syringes with needles, absorbent cotton, gauze and bandage containing blood, used ampoules and so forth. Then, the gelling material according to the invention produced as above was added, and the system was lightly agitated with a wooden spatula having a length of 20 cm and a width of 3 cm. The bag of the gelling material was disintegrated in about 10 secondes, and the system was gelled in about 45 seconds. Thus, the medical wastes could be converted into a gel (i.e., solid) in a short period of time and conveniently.

### Example 4

A bag was produced by heat sealing along three edges of two overlapped oblong pieces 5 cm × 25 cm in size cut from "Dissolvo WAL" manufactured by Mishima Paper Industries, Ltd. with a pasting space of about 1 cm left. The bag was then filled with 40 g of "SANWET IM-2200D" (absorbing capacity : 400 ml/g), an acrylic acid starch graft copolymer water-absorbing resin manufactured by Sanyo Chemical Industries, Ltd., and then its opening was heat sealed, thus obtaining a gelling material according to the invention for body fluids collected in body fluid suction equipment.

This gelling material was thrown into a liner (i.e. a disposable waste liquid container) provided in a canister fixing container of "Receptal", a medical suction system manufactured by Dainabot Ltd. Two liters of waste blood of the operated patient was collected by suction into the liner from an upper opening thereof in about 30 seconds. The bag of the gelling material was disintegrated in about 20 secondes, and the body fluid was gellated in about one minute from the start of its collection. Thus, body fluid in the form of fluid could be converted into solid in a very short period of time and conveniently.

### Comparative Example 5

A bag was produced by heat sealing along three edges of two overlapped oblong pieces 5 cm × 25 cm in size cut from "Dissolvo WA" manufactured by Mishima Paper Mfg. Co., Ltd. with a pasting space of about 1 cm left. The bag was then filled with 40 g of "SANWET IM-5000" (absorbing capacity : 400 ml/g), partially crosslinked sodium polyacrylate manufactured by Sanyo Chemical Industries, Ltd., and then its opening was heat sealed, thus obtaining a gelling material for body fluid collected by body fluid suction equipment according to the invention.

This gelling material was thrown into a liner (i.e., a disposable waste liquid container) provided in a canister fixing container of "Receptal", a medical suction system manufactured by Dainabot Ltd. Then, 2 liters of waste blood of the operated patient was collected by suction in the liner from an upper opening thereof in 30 seconds. The bag of the gelling material was disintegrated in about 10 secondes, and the body fluid was gelled in about 45 seconds from the start of its collection. Thus, body fluid in the form of liquid could be converted into solid in a short period of time and conveniently.

### Comparative Example 6

A bag was produced by heat sealing along three edges of two overlapped oblong pieces 5 cm × 25 cm in size cut from a commerically available water-soluble polyvinyl alcohol (Poval) film with a pasting space of about 1cm left: The bag was then filled with 40 g of partially cross linked sodium acrylate-acrylamide copolymer (absorbing capacity : 250 ml/g), and its opening was heat sealed, thus obtaining a gelling material for body fluid suction equipment according to the invention.

This gelling material was thrown into and set in a liner (i.e., disposable waste liquid container) provided in a canister fixing container of "Receptal", a medical suction system manufactured by Dainabot Ltd. Then, 2 liters of waste blood of the operated patient was collected by withdrawal in the liner from an upper opening thereof in 30 seconds. The bag of the gelling material was disintegrated in about 90 secondes, and the body fluid was gelled in about 3 minutes from the start of its collection. Thus, body fluid in the form of liquid could be converted to solid in a short period of time and conveniently.

The invention can provide the following various merits.
(1) The absorbent and other components are wrapped in a laminate of a water-disintegrating paper and a water-soluble film. Therefore, unlike the prior art gelling material, the gelling material according to the invention will never be spilled or dusted during handling.
   Namely, the gelling material being a water-absorbent wrapped in bags etc. which disintegrate or dissolved when being wetted, can be made gel upon being put into the aqueous fluid such as body fluid etc. together with the bags. Thus, it is very convenient in use.
(2) Unlike the prior art, the gelling material does not require times for measuring and manually sprinkling for addition to the aqueous fluid. Thus, it can be handled without taking time and conveniently.
(3) It has high absorbing capacity, and therefore it may be used in small quantities.
(4) Even if external pressure is applied after absorption, the fluid content once absorbed is never released.

Since the invention has the above effects, they are useful for processing sludge-like waste fluid produced in civil and construction sites, paper pulp plants, fiber manufacturing and drying plants, chemical plants, electric and electronic product plants and food processing plants.

Recently, blood infection of disease viruses such as aids and B-type hepatitis are constituting social problems, and safe treatment of body fluids (such as blood, anmiotic fluid and lymph fluid) produced during operation and parturition is demanded. A method is well known, for instance, where body fluid coming out during operation is recovered in a specified container with or without use of a section device and burned out for disposal. However, if the body fluid container is occasionally broken during transportation to the incineration site or body fluid is occasionally spilled by mistake, the body fluid can flow out and sink into the ground. In such case, it is difficult to recover the body fluid. If the gelling material and gelling method according to the invention is applied thereon, the liquified body fluid is solidified (by gelling), and flowing out of the liquid body fluid can be prevented even if the container is cracked. Also, if it is spilled out onto the ground, it can be easily gathered with a broom for preventing polution and ensuring safety.

In addition, if liquid body fluid is charged into the incineration furnace, the temperature in the furnace is suddenly reduced. Therefore, the fluid has to be charged in small amounts by means of spraying. This means that large-scale charging equipment is necessary and that the furnace temperature control is difficult. According to the invention, the body fluid can be charged into the furnace after it has been gelled (i.e., solidified). By so doing, like the case of burning wet wood, the furnace temperature is not reduced suddenly, permitting ready burning.

With the the above effects, the gelling material and gelling method according to the invention are useful for the treatment of medical wastes produced in hospitals, maternity hospitals, public health centers and other medical facilities.

And also, the gelling material and gelling method are particularly useful for body fluid suction equipment.

## Claims

1. Use, for gelling aqueous fluid waste in a container, of a water-absorbent comprising a resin which is in the form of powder or granules and is releasably contained in a wrapping, characterized in that the water-absorbent further comprises a filler and the wrapping comprises a laminate of a water-disintegratable paper forming the outer surface of the wrapping with a water-soluble film forming the inner surface of the wrapping.

2. Use according to claim 1 wherein said aqueous fluid waste is medical fluid waste.

3. Use according to claim 2 wherein said medical fluid waste is body fluid collected by body fluid suction equipment.

4. Use according to any of claims 1 to 3 wherein said filler is selected from the following group: activated clay, zeolite, talc, diatomaceous earth, bentonite, caryon, clay, silica gel, sand, pumice, gypsum, sawdust, pulp, paper pieces and resin chips.

5. Use according to claim 2 or claim 3 wherein the medical waste fluid which is gelled contains medical tools and solid disposables.

6. A method of gelling aqueous fluid comprising adding an aqueous fluid gelling material to aqueous fluid, wherein said aqueous fluid gelling material comprises a water-absorbent releasably contained in a wrapping comprising a laminated sheet which comprises water-disintegrating paper forming the outer surface of the wrapping and water-soluble film forming the inner surface of the wrapping, and said water-absorbent comprises a water-absorbing resin in the form of powders or granules, wherein said gelling material further contains a filler.

7. A method according to claim 6, wherein said filler is selected from the following group: activated clay, zeolite, talc, diatomaceous earth, bentonite, caryon, clay, silica gel, sand, pumice, gypsum, sawdust, pulp, paper pieces and resin chips.

8. A method according to claim 6 or claim 7 wherein said aqueous fluid is medical waste fluid.

9. A method according to claim 8, wherein said medical waste fluid is body fluid collected by body suction equipment.

10. A method according to claim 9 wherein said body fluid flows into a disposable waste container set in a canister fixing container of body fluid suction equipment.

## Patentansprüche

1. Verwendung eines Wasserabsorptionsmittels zum Gelieren von wäßrigen Abfallfluiden in einem Behälter, wobei das Wasserabsorptionsmittel ein Harz umfaßt, das in Form von Pulver oder Körnern vorliegt und freisetzbar in einer Hülle enthalten ist, dadurch gekennzeichnet, daß das Wasserabsorptionsmittel außerdem einen Füllstoff umfaßt, und die Hülle ein Laminat aus einem sich in Wasser zersetzenden Papier, das die Außenoberfläche der Hülle bildet, mit einer wasserlöslichen Folie, die die Innenoberfläche der Hülle bildet, umfaßt.

2. Verwendung nach Anspruch 1, wobei das wäßrige Abfallfluid ein medizinisches Abfallfluid ist.

3. Verwendung nach Anspruch 2, wobei das medizinische Abfallfluid Körperflüssigkeit ist, die von einer Absaugvorrichtung für Körperflüssigkeiten aufgefangen wird.

4. Verwendung nach einem der Ansprüche 1 bis 3, wobei der Füllstoff aus der folgenden Gruppe ausgewählt ist: aktivierter Ton, Zeolith, Talkum, Kieselerde, Bentonit, Caryon, Ton, Kieselgel, Sand, Bimsstein, Gips, Sägemehl, Zellstoff, Papierschnitzel und Harzstücke.

5. Verwendung nach Anspruch 2 oder Anspruch 3, wobei das medizinische Abfallfluid, das geliert ist, medizinische Instrumente und feste Einwegartikel enthält.

6. Verfahren zum Gelieren eines wäßrigen Fluids, das das Zusetzen eines das wäßrige Fluid gelierenden Mittels zum wäßrigen Fluid umfaßt, wobei das das wäßrige Fluid gelierende Mittel ein Wasserabsorptionsmittel umfaßt, das freisetzbar in einer Hülle enthalten ist, die eine laminierte Lage umfaßt, die sich in Wasser zersetzendes Papier, das die Außenoberfläche der Hülle bildet, und wasserlösliche Folie, die die Innenoberfläche der Hülle bildet, umfaßt, und wobei das Wasserabsorptionsmittel ein Wasser absorbierendes Harz in Form von Pulver oder Körnern umfaßt, wobei das Geliermittel außerdem einen Füllstoff enthält.

7. Verfahren nach Anspruch 6, wobei der Füllstoff aus der folgenden Gruppe ausgewählt ist: aktivierter Ton, Zeolith, Talkum, Kieselerde, Bentonit, Caryon, Ton, Kieselgel, Sand, Bimsstein, Gips, Sägemehl, Zellstoff, Papierschnitzel und Harzstücke.

8. Verfahren nach Anspruch 6 oder Anspruch 7, wobei das wäßrige Fluid medizinisches Abfallfluid ist.

9. Verfahren nach Anspruch 8, wobei das medizinische Abfallfluid Körperflüssigkeit ist, die von einer Absaugvorrichtung für Körperflüssigkeiten aufgefangen wird.

10. Verfahren nach Anspruch 9, wobei die Körperflüssigkeit in einen Behälter für entsorgbare Abfälle strömt, der in einen den Kanister fest aufnehmenden Behälter einer Absaugvorrichtung für Körperflüssigkeiten eingesetzt ist.

## Revendications

1. Utilisation, pour la gélification de déchets fluides aqueux dans un récipient, d'une matière absorbant l'eau comprenant une résine qui se présente sous la forme de poudres ou de granulés et est contenue d'une manière libérable dans un conditionnement, caractérisée en ce que la matière absorbant l'eau comprend par ailleurs une charge et le conditionnement comprend un stratifié de papier désintégrable dans l'eau formant la surface extérieure du conditionnement avec un film soluble dans l'eau formant la surface intérieure du conditionnement.

2. Utilisation selon la revendication 1, dans laquelle ledit déchet fluide aqueux est un déchet fluide médical.

3. Utilisation selon la revendication 2, dans laquelle ledit déchet fluide médical est un fluide corporel recueilli par un appareil d'aspiration de fluides corporels.

4. Utilisation selon l'une quelconque des revendications 1 à 3, dans laquelle ladite charge est sélectionnée dans le groupe suivant : l'argile activé, les zéolites, le talc, la terre à diatomées, la bentonite, le caryon, l'argile, le gel de silice, le sable, la pierre ponce, le gypse, la sciure, la pâte à papier, les fragments de papier et des pastilles de résine.

5. Utilisation selon la revendication 2 ou 3, dans laquelle le déchet fluide médical qui est gélifié contient des instruments et des disposables solides médicaux.

6. Procédé de gélification de fluide aqueux comprenant l'addition d'une matière gélifiante à un fluide aqueux, ladite matière gélifiante comprenant une matière absorbant l'eau contenue d'une manière libérable dans un conditionnement comprenant une feuille stratifiée qui comprend du papier qui se désintègre à l'eau formant la surface extérieure du conditionnement et un film soluble dans l'eau formant la surface intérieure du conditionnement et ladite matière absorbant l'eau comprenant une résine absorbant l'eau sous la forme de poudres ou de granulés, dans lequel ladite matière gélifiante contient par ailleurs une charge.

7. Procédé selon la revendication 6, dans lequel ladite charge est sélectionnée dans le groupe suivant : l'argile activé, les zéolites, le talc, la terre à diatomées, la bentonite, le caryon, l'argile, le gel de silice, le sable, la pierre ponce, le gypse, la sciure, la pâte à papier, les fragments de papier et des pastilles de résine.

8. Procédé selon la revendication 6 ou 7, dans lequel ledit fluide aqueux est un déchet médical fluide.

9. Procédé selon la revendication 8, dans lequel ledit déchet médical fluide est un fluide corporel recueilli par un appareil d'aspiration de fluides corporels.

10. Procédé selon la revendication 9, dans lequel ledit fluide corporel s'écoule dans un récipient de déchets disposables installé dans une boîte recevant le récipient dudit appareil d'aspiration de fluides corporels.
